# EUROPEAN PATENT SPECIFICATION

(11) **EP 3 908 191 B1**
(45) Date of publication and mention of the grant of the patent: **10.04.2024**
(21) Application number: 19829241.9
(22) Date of filing: 30.12.2019
(51) Int. Cl.: A61B 8/08

(54) **ULTRASOUND SYSTEM AND METHOD FOR SHEAR WAVE CHARACTERIZATION OF ANISOTROPIC TISSUE**
ULTRASCHALLSYSTEM UND VERFAHREN ZUR KARAKTERISIERUNG ANISOTROPES GEWEBES MITTELS SCHERWELLEN
SYSTÈME À ULTRASONS POUR LA CARACTÉRISATION D'UN TISSU ANISOTROPE À L'AIDE D'ONDES DE CISAILLEMENT

(30) Priority: 09.01.2019 US 201962790187 P
(43) Date of publication of application: 17.11.2021
(73) Proprietor: Koninklijke Philips N.V., 5656 AG Eindhoven (NL)
(72) Inventor: AMADOR CARRASCAL, Carolina, 5656 AE Eindhoven (NL); VIGNON, Francois Guy Gerard Marie, 5656 AE Eindhoven (NL); KIM, Seungsoo, 5656 AE Eindhoven (NL); XIE, Hua, 5656 AE Eindhoven (NL)
(74) Representative: Philips Intellectual Property & Standards
(86) International application number: PCT/EP2019/087135
(87) International publication number: WO 2020/144077

(56) References cited:
- WEI-NING LEE ET AL: "Noninvasive assessment of myocardial anisotropy in vitro and in vivo using Supersonic Shear Wave Imaging", ULTRASONICS SYMPOSIUM (IUS), 2010 IEEE, IEEE, 11 October 2010 (2010-10-11), pages 690-693, XP031953033, DOI: 10.1109/ULTSYM.2010.5935898 ISBN: 978-1-4577-0382-9
- E. I. MADARAS ET AL: "Anisotropy of the ultrasonic backscatter of myocardial tissue: II. Measurements i n v i v o", THE JOURNAL OF THE ACOUSTICAL SOCIETY OF AMERICA, vol. 83, no. 2, 1 February 1988 (1988-02-01), pages 762-769, XP055679149, New York, NY, US ISSN: 0001-4966, DOI: 10.1121/1.396119

## Description

### TECHNICAL FIELD

The present disclosure pertains to ultrasound systems and methods for imaging anisotropic tissue such as cardiac tissue, and particularly systems which improve the scanning protocol and user interface of a system configured to perform shear wave elastography (SWE).

### BACKGROUND

An ultrasound imaging system, such as a cart-based ultrasound imaging system, typically includes a user interface, which operates in conjunction with a probe and a display to acquire and display images from a subject, such as a patient. The ultrasound imaging system may use shear wave elastography to determine mechanical properties of tissue. Shear wave elastography generally involves the process of applying a force (acoustically or mechanically) in a given region of biological tissue and monitoring the propagation of shear waves through the tissue to determine the properties of the tissue (e.g., tissue stiffness). Shear wave elastography may thus be used for screening and diagnostic purposes such as to identify regions of abnormal stiffness in tissues, which may indicate the presence of for example, a tumor.

Different types of tissue have different properties. Certain types of tissue, such as liver tissue, are generally isotropic, that is, properties of the tissue are the same in all directions. Certain other types of tissue, e.g., musculoskeletal, vascular wall, and myocardium tissue, are anisotropic, where a property of the tissue (e.g., stiffness) may vary based on a direction along which that property is measured. The anisotropy of a tissue may be based on the orientation of fibers within that tissue. Complex anisotropic tissue, such as cardiac tissue, may have fibers which change orientation throughout the tissue, leading to complex anisotropic properties. Thus, conventional techniques for shear wave elastography, which may base a tissue stiffness determination on a single measurement at an indiscriminately selected image plane, may be inadequate for characterizing complex anisotropic tissue, such as cardiac tissue. Thus, designers and manufacturers of ultrasound imaging systems continue to seek improvements to shear wave elastography system used for imaging and characterizing anisotropic tissue.

The publications by Wei-Ning Lee et al.: "Noninvasive assessment of myocardial anisotropy in vitro and in vivo using Supersonic Shear Wave Imaging", ULTRASONICS SYMPOSIUM, 11 October 2010, pages 690-693 and by E. I. Madaras et al.: "Anisotropy of the ultrasonic backscatter of myocardial tissue: II. Measurements in vivo", THE JOURNAL OF THE ACOUSTICAL SOCIETY OF AMERICA, vol. 83, no. 2, 1 February 1988, pages 762-769 show two analytical methods for investigating the orientation of myocardial fibers.

### SUMMARY OF THE INVENTION

The aspects of the invention are defined in the independent claims. Further advantageous embodiments are defined in the dependent claims.

### SUMMARY OF THE DISCLOSURE

The systems and methods described herein may, in some applications, improve consistency and/or reliability of SWE measurements in anisotropic tissue. In some embodiments, the systems and methods may provide for ways of characterizing the anisotropy of tissues.

As described herein, an initial set of measurements may be acquired at a variety of imaging planes having different orientations with respect to the tissue. The initial measurements may be used to determine the orientation of structures (e.g., fibers) within the tissue. The initial measurements may be used to select imaging planes that are at desired orientations to the structures of the tissue (e.g., aligned, orthogonal). Shear waves may be induced at the intersection of the selected imaging planes. To acquire SWE measurements, the shear waves may be tracked along the selected imaging planes. The SWE measurements along each of the selected imaging planes may be provided and/or used to generate a composite SWE measurement. By using the initial measurements to select the planes for tracking the shear waves, more consistent and/or reliable SWE measurements may be acquired. Combining and/or comparing the SWE measurements along the different selected imaging planes may provide a method of characterizing anisotropy in tissue.

According to embodiments of the disclosure, a method of acquiring shear wave elastography measurements of anisotropic tissue may include acquiring initial measurements from the anisotropic tissue by transmitting ultrasound beams toward the anisotropic tissue at a plurality of different angles with respect to an orientation of the anisotropic tissue, determining a first imaging plane at the angle associated with a maximum or a minimum value of the initial acoustic measurements, wherein the maximum value indicates a first orientation to a structure of the anisotropic tissue and the minimum value indicates a second orientation to the structure of the anisotropic tissue, determining a second imaging plane, generating a first shear wave at an intersection of the first imaging plane and the second imaging plane, acquiring a first shear wave elastography measurement by tracking the first shear wave propagation along the first imaging plane, generating a second shear wave at the intersection of the first imaging plane and the second imaging plane, acquiring a second shear wave elastography measurement by tracking the second shear wave propagation along the second imaging plane, and generating a composite shear wave elastography measurement for the anisotropic tissue at the intersection of the first imaging plane and the second imaging plane based on the first and second shear wave elastography measurements.

According to embodiments of the disclosure, an ultrasound system may include a probe configured to transmit ultrasound signals and acquire echoes responsive to the ultrasound signals to acquire measurements from an imaging plane and a processor. The processor may be configured to cause the probe to acquire initial measurements from an anisotropic tissue at a plurality of angles with respect to an orientation of the anisotropic tissue, determine a first imaging plane at an angle associated with a maximum or minimum value of the initial measurements, wherein the maximum value indicates a first orientation to a structure of the anisotropic tissue and the minimum value indicates a second orientation to the structure of the anisotropic tissue, determine a second imaging plane, cause the probe to generate a first shear wave at an intersection of the first imaging plane and the second imaging plane, acquire a first shear wave elastography measurement at the intersection of the first imaging plane and the second imaging plane by causing the probe to track the first shear wave's propagation along the first imaging plane, cause the probe to generate a second shear wave at the intersection of the first imaging plane and the second imaging plane, acquire a second shear wave elastography measurement at the intersection of the first imaging plane and the second imaging plane by causing the probe to track the second shear wave's propagation along the second imaging plane, and generate a composite shear wave elastography measurement anisotropic tissue at the intersection of the first imaging plane and the second imaging plane based on the first and second shear wave elastography measurements.

### BRIEF DESCRIPTION OF THE DRAWINGS

Figure 1 is a block diagram of an operational environment for an ultrasound system in accordance with some examples of the present disclosure.
Figure 2 is a block diagram of an ultrasound system in accordance with some examples of the present disclosure.
Figure 3 is a block diagram of a method of collecting shear wave elastography measurements from complex anisotropic tissue in accordance with some examples of the present disclosure.
Figure 4 is a block diagram depicting an example display of an ultrasound system in accordance with some examples of the present disclosure.
Figure 5 is an example report generated by an ultrasound system in accordance with some examples of the present disclosure.

### DETAILED DESCRIPTION

The following description of certain embodiments is merely exemplary in nature and is in no way intended to limit the invention or its applications or uses. In the following detailed description of embodiments of the present systems and methods, reference is made to the accompanying drawings which form a part hereof, and which are shown by way of illustration specific embodiments in which the described systems and methods may be practiced. These embodiments are described in sufficient detail to enable those skilled in the art to practice presently disclosed systems and methods, and it is to be understood that other embodiments may be utilized and that structural and logical changes may be made without departing from the scope of the present system. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

The present technology is also described below with reference to block diagrams and/or flowchart illustrations of methods, apparatus (systems) and/or computer program products according to the present embodiments. It is understood that blocks of the block diagrams and/or flowchart illustrations, and combinations of blocks in the block diagrams and/or flowchart illustrations, may be implemented by computer executable instructions. These computer executable instructions may be provided to a processor, controller or controlling unit of a general purpose computer, special purpose computer, and/or other programmable data processing apparatus to produce a machine, such that the instructions, which execute via the processor of the computer and/or other programmable data processing apparatus, create means for implementing the functions/acts specified in the block diagrams and/or flowchart block or blocks.

Ultrasound shear wave elastography (SWE) conventionally assumes that shear waves propagate in isotropic materials, in other words the material mechanical properties are the same in all directions. Consequently, translating SWE to anisotropic media such as the myocardium is challenging due to the complex shear wave propagation in complex myocardium architecture. Compared to musculoskeletal tissue, which may be approximated as a transverse isotropic material (two axis in which the mechanical properties are the same and one axis where the mechanical properties vary), myocardium architecture is known to be complex with fiber orientation varying continuously throughout the heart walls. Preliminary studies in cardiac elastography have reported faster shear wave speed measured through a short axis view and slower shear wave speed measured through a long axis view. Cardiac elastography is gaining interest in the medical imaging community but so far has limited clinical acceptance due to the lack of understanding of shear wave propagation in complex myocardium architecture and the lack of a uniform scanning protocol to ensure robust and reproducible measurements that are not biased by fiber and probe orientation.

Systems and methods according to the present disclosure may address one or more problems in the area of SWE, particularly as applied to complex anisotropic tissue, such as myocardium tissue. For example, in accordance with the principles of the present disclosure, an ultrasound imaging system may be provided with a cardiac shear wave imaging mode which improves (e.g., provides user guidance and/or at least partially automates steps in the scanning protocol) the acquisition of shear wave elastography data for cardiac tissue. As described further below, when performing shear wave elastography with anisotropic tissue, it may be desirable to acquire SWE measurements at different orthogonal intersecting planes (e.g., a pair of orthogonal planes) and in some cases at multiple sets of intersecting planes for more robust characterization of the tissue. However, it may be difficult for an operator, particularly inexperienced operators, to execute the appropriate scanning sequence and/or to precisely control the placement or orientation of the imaging plane of the probe with respect to the tissue to obtain the appropriate measurements.

An apparatus, system, and method in accordance with embodiments of the present disclosure may provide technical solutions for more easily acquiring appropriate shear wave elastography measurements of anisotropic tissue, particularly complex anisotropic tissue such as the myocardium, to assist a particular clinical purpose (e.g., disease progression monitoring or diagnosis). In some examples, a scan protocol and user interface may be provided to guide acquisition of orthogonal measurements at a variety of locations about the tissue. Initial measurements (e.g., via an initial ultrasound scan of the tissue) may be used to determine a scan orientation (e.g., imaging plane) that is aligned with tissue structures (e.g., muscle fibers) and a scan orientation that is orthogonal to the tissue structures. The scan orientations aligned with and orthogonal to the tissue structures may be used as first and second scan orientations at a given location of the probe with respect to the tissue. In some examples, feedback may be provided to the user (e.g., with a user interface on a display) to guide the acquisition of shear wave elastography measurements at the first and second scan orientations. In some embodiments, the first scan orientation may align with a short axis and second scan orientation may align with a long axis of fibers of the anisotropic tissue at the current location.

Determination of the first and second scan orientations and acquisition of SWE measurements may be repeated at multiple locations of interest about the complex anisotropic tissue, e.g. by moving the probe at different locations with respect to the tissue. In some examples, a 3D imaging probe may be used and in some such cases, obtaining measurements at different locations about the tissue may not require moving the probe relative to the tissue as the probe may be capable (e.g., through electronic scanning) to scan at multiple different imaging planes through a volume.

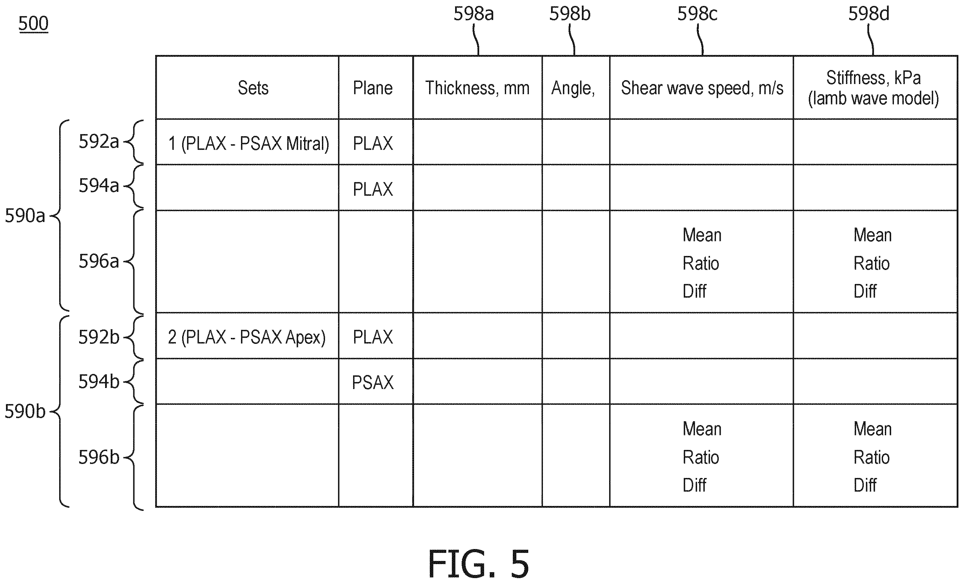

In some examples of the present disclosure, the system may also generate a measurement report based on the shear wave elastography measurements obtained in accordance with the scan protocol(s) described herein. In some examples, the SWE measurements (e.g., shear wave speed, wall thickness, wall angle (orientation), measurement orientation, and/or stiffness) may be tabulated, and in some cases a reported SWE measurement (e.g., a SWE measurement included in the report and/or displayed to the user) may be a composite SWE measurement produced based upon multiple individual SWE measurements, such as a pair of SWE measurements obtained at the first scan orientation and the second scan orientation at one of the locations of interest (e.g., as shown in the report in Figure 5). While examples are described herein in the context of complex anisotropic tissue, it will be understood that the principles of the present disclosures may be applied to any type of anisotropic tissue (e.g., muscle tissue/fibers extending substantially in one direction).

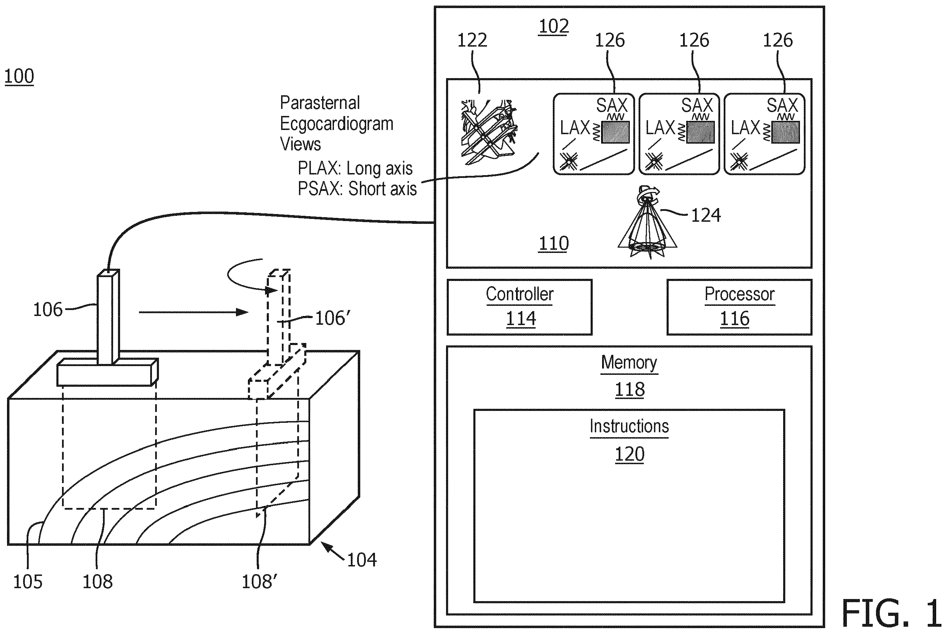

Figure 1 depicts an operational environment 100 associated with an ultrasound system 102 according to some embodiments of the present disclosure. Components of the operational environment 100 may be used, at least in part, to implement embodiments of the present disclosure. Shown in Figure 1 are an ultrasound system 102 and a probe 106 communicatively coupled (e.g., via a wired or wireless connection) to the ultrasound system 102. The probe 106 may collect data from an imaging plane 108 which may be positioned to capture data from a region including fibers 105 of anisotropic tissue 104. The ultrasound system 102 may include a display 110, a controller 114, a processor 116, and a memory 118 including instructions 120. The display 110 may produce one or more graphics, such as a location feedback display 122, an orientation feedback display 124, and/or image display(s) 126.

The probe 106 may be a handheld unit coupled to the ultrasound system 102. The probe includes an array of ultrasound transducer elements, which may selectively generate and transmit ultrasound signals (e.g., toward biological tissue) and detect echoes from the transmitted ultrasound signals. The probe 106 is configured to acquire echoes from a plurality of A-lines for generating two-dimensional images at a given imaging plane 108. A position of the imaging plane 108 may be defined based on the orientation of the probe 106. Some probes may be configured to image tissue along a single imaging plane, or the transmission and acquisition of ultrasound signals may be controllable (e.g., by mechanical scanning of the array within the probe or by electronic steering of beams produced by a 2D array) to image at different imaging planes 108. During operation of the system, the probe may be positioned against an acoustic window (e.g., against the skin of the subject, which may be coated with acoustic coupling gel) near a region of anisotropic tissue 104. For purposes of illustration, the anisotropic tissue 104 is shown in close proximity to the probe 106, however in practice there may be one or more additional layers or types of tissue (e.g., bone, skin, fat, muscle, etc.) between the probe 106 and the anisotropic tissue 104 being imaged. When the probe 106 is positioned with respect to the acoustic window, the probe 106 can be operated to acquire image data in the imaging plane 108 which extends through the biological tissue intersecting the biological tissue of interest (e.g., anisotropic tissue 104).

The probe 106, and specifically the activation of elements of the array for transmitting and receiving ultrasound, may be controlled by the ultrasound system 102 to acquire image data (e.g., for obtaining measurements and/or producing ultrasound images, such as 2D images) of the anisotropic tissue 104 in the imaging plane 108. Although shown in Figure 1 as rectangular, it is understood by those of skill in the art that the imaging plane 108 may have a different configuration such as, for example, curvilinear, trapezoidal, sector, and/or radial, e.g., depending upon the probe used and/or multiplanar reformatting of the image data. In some embodiments, the probe 106 may record measurements from a plurality of imaging planes, which may intersect the tissue 104 at different angles. The plurality of imaging planes may be fixed with relation to the probe 106, or may be steerable (e.g., with beam steering) such that the plurality of imaging planes can be 'swept' through a tissue of interest. The ultrasound system 104 may produce a 2D or 3D image based on the plurality of imaging planes. In some embodiments, the probe 106 may include a 2D array of transducers and may be able to selectively generate one or more imaging planes 108 at a plurality of orientations with respect to the probe 106 (e.g., the planes may be at different angles with respect to a face of the 2D array as well as at different angles with respect to a normal through the face of the 2D array).

The probe 106 may be used to acquire a shear wave elastography measurements of the anisotropic tissue 104. To that end, the probe 106 may include a transducer which is operable to transmit a "push pulse" toward the anisotropic tissue 104, generating a shear wave which then propagates through the anisotropic tissue 104. Alternatively, the shear wave in the tissue may be generated without acoustic radiation force but via mechanical force applied externally to the tissue, such as by a mechanical vibrator configured to compress the tissue. The probe 106 may be further operable to emit tracking pulses, which may be used to measure (or track) the velocity of the shear wave as it propagates. The measured velocity of the shear wave may be analyzed (such as by processor 116) to determine a stiffness of the anisotropic tissue 104. In some embodiments, the stiffness may be determined from the velocity of the shear wave using a Lamb wave model. The shear wave elastography data may be used to produce a shear wave elastography image.

The anisotropic tissue 104 may have anisotropic properties (e.g., stiffness) which vary based on the orientation of fibers 105 in the tissue 104. In some complex tissues, such as myocardial tissue, the fibers 105 may change orientation multiple times along their length, and there may be multiple layers of fibers 105 at different orientations at a given location of the tissue 104. Because the anisotropic tissue 104 has complex changes in the fiber 105 measurements acquired at orientation imaging planes at two different locations may record different stiffness values, even if the imaging planes have the same orientation with respect to the tissue 104. Thus, multiple measurements may need to be taken at multiple orientations and locations to characterize anisotropic properties of the tissue 104.

In the operational environment 100, the probe 106 is depicted as moving from a first position in which the probe is associated with an imaging plane 108 to a second position (with the probe 106' shown in phantom line), in which the probe is associated an imaging plane 108'. As shown by the arrows, imaging at the imaging plane 108' may involve moving the probe to a different position with respect to the tissue or it may involve steering the ultrasound beams to a different plane through the tissue. A change in the position of the probe may involve re-positioning of the probe to a different location (e.g., a translation of the probe such that the probe is in contact with a different portion of the skin of the subject), a different orientation (e.g., a tilting or toe-heeling of the probe while maintaining the probe at the same location on the skin), or a combination of the two. As shown in Figure 1, it may be necessary to physically move and/or rotate the probe 106 to acquire image data at a different imaging plane 108. In other examples, imaging at different planes may be achieved without moving the probe 106, (e.g., with beam steering). While a change in rotation is shown as a rotation of the imaging plane 108 about an axis normal to a surface of the tissue 104, it is to be understood that a change in orientation may involve rotation about any axis, and may include rotation about multiple axes. In some embodiments the probe 106 may be tilted to change an orientation of the imaging plane 108.

The ultrasound system 102 may direct placement of the probe 106 at different locations about the anisotropic tissue 104. The ultrasound system 102 may direct initial measurements of tissue properties at each of the different locations. The system 102 may direct a user to collect the initial measurements, or may automatically collect the initial measurements. The initial measurements may be acoustic and/or mechanical properties of the tissue 104 which may be used to determine the orientation of the fibers 105 with respect to the imaging plane 108. The initial measurements may be shear wave elastography imaging, or may be a different form of ultrasound imaging, such as measuring backscattering or B-mode imaging. In some embodiments, the initial measurements may be acoustic attenuation, speed of sound, tissue motion, shear wave speed, relative stiffness and/or other measurement modalities. The initial measurements may be dependent on the angle of the imaging plane 106 along which the initial measurement were collected with respect to the tissue 104. As an example, when the initial measurement is a backscattering measurement, the probe 106 may record a maximum backscattering value when the initial measurements are collected at an angle perpendicular to the long axis of the fibers 105, and a minimum value when the imaging plane 108 is at an angle parallel to the orientation of the fibers 105.

Based on the initial measurements, the system 102 may determine a first imaging orientation and a second imaging orientation based on the initial measurements and may direct shear wave elastography measurements at the first and second imaging orientation. A user may be prompted to collect the shear wave elastography measurements, or the system may collect them automatically. The first orientation and the second orientation may be orthogonal to each other. In some embodiments, the first orientation may be aligned with an axis of the fibers 105 (e.g., along a long axis of the fibers 105) at the current location of the probe 106, while the second orientation may be orthogonal to an axis of the fibers 105 (e.g., along a short axis of the fibers 105). Once the shear wave measurements are collected, the ultrasound system 102 may direct placement of the probe 106 at a different location on the tissue. The process of collecting initial measurements, determining image orientations, and collecting shear wave measurements may then repeat at the different location. By directing the placement of the probe 106 at different locations of the tissue 104 and directing collection of multiple (e.g., orthogonal) shear wave measurements at each location, the ultrasound system 102 may characterize anisotropic properties of the tissue 104.

The ultrasound system 102 is coupled to the probe 106 to receive and process data therefrom and to direct operation of the probe 106. The ultrasound system 102 may be directly coupled to the probe 106 (e.g., with a cable), or may be coupled via a wireless connection (e.g., Wi-Fi, Bluetooth). The ultrasound system 102 may include a controller 114 to direct operation of the probe 106. The ultrasound system 102 may include a memory 118 which may hold instructions 120. The instructions 120 may include processor-executable instructions, which may be executed by a processor of the system (e.g., processor 116) to cause the controller 114 to direct the probe 106 to operate in specific ways. The instruction 120 may also include executable instruction configured to cause the processor 116 to read and/or analyze data from the probe and/or to produce feedback (e.g., operator guidance) for a user of the ultrasound system 102 and/or reports, which may be used for clinical purpose (e.g., diagnosis).

The ultrasound system 102 may include a display 110 configured to provide data (e.g., image data displayed in the form of an ultrasound image of desired format) and/or feedback (e.g., operator guidance) to a user of the ultrasound system 102. The display 110 may be configured to display, responsive to a processor of the system, one or more graphics, which may be configured to provide guidance to an operator of the ultrasound system 102 or which may provide results of the imaging session. For example, the display 110 may provide a graphical user interface such as a location feedback display 122 which may instruct the use to place the probe 106 at one or more different locations with respect to the tissue 104. In some examples, the display 110 may include a graphical user interface such as an orientation display to direct placement of the probe 106 at different orientations with respect to the tissue 104. The display 110 may display one or more images 126 which may be real-time images from the imaging plane 108, and/or may be images saved to the memory 118. The display may also show one or more reports, which may summarize measurements collected by the ultrasound system 102 and stored in the memory 118. For example, the report may include composite SWE measurements (see e.g., Figure 5) generated from the SWE measurements at multiple imaging planes at a given location of interest. In some examples the composite SWE measurements may be generated from SWE measurements at multiple locations of interest.

The location feedback display 122 and/or the orientation feedback display 124 may be used to direct positioning of the imaging plane 108 in the tissue. In some embodiments, a user may manually adjust a location and/or orientation of the probe 106 based on the location feedback display 122 and orientation feedback display 124 such as to achieve data acquisition at a desired imaging plane as instructed by the location feedback display 122.

In some embodiments, the location and/or orientation of the imaging plane 108 may be automatically adjusted by the ultrasound system 102. For example, as described, the probe 106 may include a 2D array of transducer elements and thus may be capable of electronic steering of the beams. The ultrasound system 120 may be configured to obtain a preliminary measurement of the tissue 104 and to determine, from the preliminary measurements, a pose or orientation of a target organ (e.g., the heart) in the tissue. For example, the system may be configured to perform an initial 3D scan of the tissue to obtain a first 3D data set, which may include for example echo intensity information (or backscatter data). The ultrasound system 102 may process the 3D dataset to determine the pose and orientation of the organ represented in the 3D dataset for example fitting the acquired data to an anatomical model of the organ being images or via another imaging processing technique.

Continuing with the example of cardiac imaging, the ultrasound system 102 may then identify a first and second target imaging planes, which may correspond to standard cardiac views such as long axis parasternal, short axis parasternal, 2-, 3- or 4-chamber apical views, or subcostal view, and generate, based on the determined pose and the controller 114, commands to adjust the operation of the probe 106 (e.g., by steering the beams to the appropriate directions) to selectively obtain shear wave measurements at the first and second imaging plane. In some examples the first and second target imaging planes may be selected at angles and locations of interest such that the first imaging plane(s) correspond to the parasternal long axis view and the second imaging plane(s) correspond to one or more of the parasternal short axis aorta view, the parasternal short axis mitral view, and/or the parasternal short axis apex view. In some embodiments the locations of interest corresponding to the intersections between these imaging planes may be selected automatically by the ultrasound system 102. In some embodiments the locations of interest may be selected manually based on, for example, anatomical knowledge.

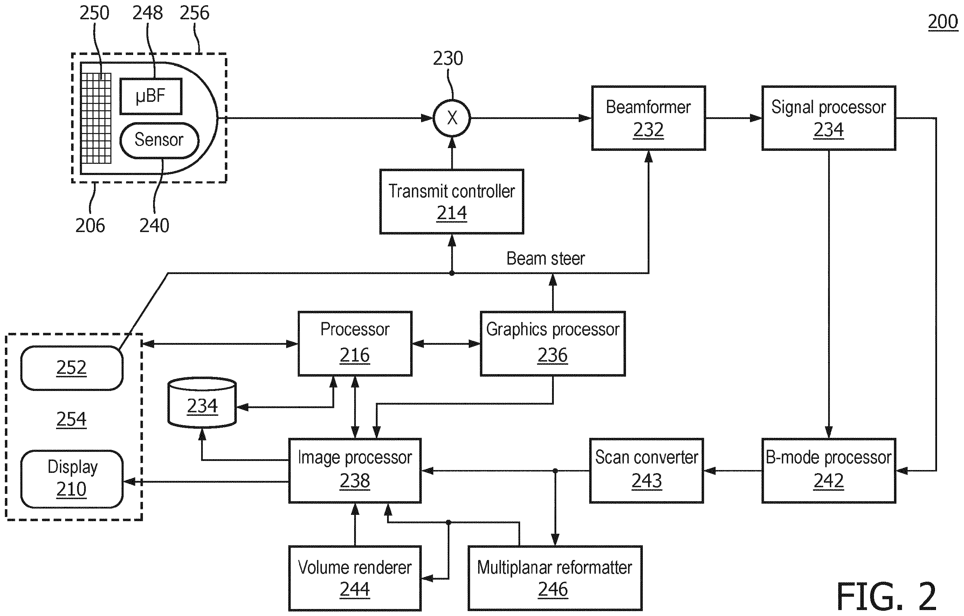

Figure 2 shows a block diagram of an ultrasound imaging system 200 according to some embodiments of the present disclosure. The ultrasound imaging system 200 may be used to implement, at least in part, the ultrasound system 102 of Figure 1. Figure 2 shows an ultrasound imaging system 200, which includes a handheld unit 256, which may include an ultrasound probe 206, a transducer array 250, microbeamformer 248, and one or more sensors 240. The ultrasound system 200 may also include a transmit/receive (T/R) switch 230, beamformer 232, transmit controller 214, signal processor 234, B-mode processor 242, scan converter 243, multiplanar reformatter 246, volume renderer 244, image processor 238, graphics processor 236, user interface 254, input device 252, and output device 210. The components shown in Figure 2 are merely illustrative, and other variations, including eliminating components, combining components, rearranging components, and substituting components are all contemplated.

The ultrasound imaging system 200 includes a probe 206, which may be used to implement the probe 106 of Figure 1 in some embodiments. The probe 206 is positioned about a subject and used to capture data about tissues of the subject. In the ultrasound imaging system 200 in Figure 2, the ultrasound probe 206 includes a transducer array 250 for transmitting ultrasonic waves and receiving echo information. A variety of transducer arrays are well known in the art, e.g., linear arrays, convex arrays or phased arrays. The transducer array 250 for example, can include a two dimensional array of transducer elements capable of scanning in both elevation and azimuth dimensions for 2D and/or 3D imaging. The transducer array 250 is coupled to a microbeamformer 248, typically located in the ultrasound probe 206, which controls transmission and reception of signals by the transducer elements in the array. In this example, the microbeamformer 248 is coupled, such as by a probe cable or wirelessly, to a transmit/receive T/R switch 230, which switches between transmission and reception. The T/R switch 230 may thus protect the beamformer 232 from high energy transmit signals. In some embodiments, the T/R switch 230 and other elements of the system can be included in the transducer probe 206 rather than in a separate ultrasound system base (e.g., ultrasound system 102 of Figure 1).

The transmission of ultrasonic beams from the transducer array 250 under control of the microbeamformer 248 is directed by the transmit controller 214 coupled to the T/R switch 230 and the beamformer 232. The transmit controller 214 receives input from the user's operation of an input device 252 of user interface 254. The transmit controller 214 may be a component of an ultrasound system base, or may be a general controller of the ultrasound system (e.g., controller 114 of Figure 1). The user interface 254 may be implemented using one or more input, such as control panels, which may include soft and/or hard controls, and output devices, such as one or more displays (e.g., display 110 of Figure 1), as described further below. One of the functions controlled by the transmit controller 214 is the direction in which beams are steered. Beams may be steered straight ahead from (orthogonal to) the transducer array, or at different angles for a wider field of view. The partially beamformed signals produced by the microbeamformer 248 are coupled to a beamformer 232 where partially beamformed signals from individual patches of transducer elements are combined into a fully beamformed signal. The transmit controller 214 may record a position of the beams with respect to the probe 206. As described here, the position of the beams and the probe 206 may be used to determine a position of an imaging plane (e.g., imaging plane 108 of Figure 1).

The beamformed signals may be coupled to a signal processor 234. The signal processor 234 can process the received echo signals in various ways, such as bandpass filtering, decimation, I and Q component separation, and harmonic signal separation. The signal processor 234 may also perform additional signal enhancement such as speckle reduction, signal compounding, and noise elimination. The processed signals may be coupled to a B-mode processor 242, which can employ amplitude detection for the imaging of structures in the body. The signals produced by the B-mode processor may be coupled to a scan converter 243 and a multiplanar reformatter 246. The scan converter 243 arranges the echo signals in the spatial relationship from which they were received in a desired image format. For instance, the scan converter 243 may arrange the echo signal into a two dimensional (2D) sector-shaped format, or a pyramidal three dimensional (3D) image. The multiplanar reformatter 246 can convert echoes, which are received from points in a common plane in a volumetric region of the body into an ultrasonic image of that plane, as described in U.S. Pat. No. 6,443,896 (Detmer). A volume renderer 244 converts the echo signals of a 3D data set into a projected 3D image as viewed from a given reference point, e.g., as described in U.S. Pat. No. 6,530,885 (Entrekin et al.) The 2D or 3D images may be coupled from the scan converter 243, multiplanar reformatter 246, and volume renderer 244 to an image processor 238 for further enhancement, buffering and temporary storage for display on an output device 210. The output device 210 may include a display device implemented using a variety of known display technologies, such as LCD, LED, OLED, or plasma display technology. In some embodiments, the output device 210 may implement the display 110 of Figure 1.

The graphics processor 236 can generate graphic overlays for display with the ultrasound images. These graphic overlays can contain, e.g., standard identifying information such as patient name, date and time of the image, imaging parameters, and the like. The graphics processor 236 may receive input, such as a typed patient name, from the input device 252. The graphics processor may generate one or more displays based on data from the probe 206, such as the location feedback display 122, orientation feedback display 124, and image display 126 of Figure 1. The input device 252 may include one or more mechanical controls, such as buttons, dials, a trackball, a physical keyboard, and others, which may also be referred to herein as hard controls. Alternatively or additionally, the input device 252 may include one or more soft controls, such as buttons, menus, soft keyboard, and other user interface control elements implemented for example using touch-sensitive technology (e.g., resistive, capacitive, or optical touch screens). To that end, the ultrasound imaging system 200 may include a user interface processor (i.e., processor 216), which may control operations of the user interface such as functions associated with soft controls. One or more of the user controls may be co-located on a control panel. For example, one or more of the mechanical controls may be provided on a console and/or one or more soft controls may be co-located on a touch screen, which may be attached to or integral with the console. For example, in some embodiments the input device 252 may be part of the display 110 of Figure 1.

The ultrasound images and associated graphics overlays may be stored in memory 218, for example for off-line analysis. In addition, the memory 218 may store processor-executable instructions including instructions for performing functions associated with the user interface 254. In some embodiments, the user interface 254 may include a graphical user interface, which may be configured to display, responsive to a processor of the system 200, graphical user interface elements for providing guidance to the sonographer in performing shear wave elastography of anisotropic tissue in accordance with any of the examples herein. The memory 218 may be a part of an ultrasound base unit, or may be a general memory that is part of a computer system coupled to the base unit (e.g., the memory 218 may be memory 118 of ultrasound system 102 of Figure 1). The user interface 254 can also be coupled to the multiplanar reformatter 246 for selection and control of a display of multiple multiplanar reformatted (MPR) images. In some examples, functionality of two or more of the processing components (e.g., beamformer 232, signal processor 234, B-mode processor 242, scan converter 243, multiplanar reformatter 246, volume renderer 244, image processor 238, graphics processor 236, processor 216, etc.) may be combined into a single processing unit such as processor 116 of Figure 1.

The probe 206, sensor 240, microbeamformer 248, and transducer 250 may be combined into a handheld unit 256. The handheld unit 256 may be shaped to be held in a user's hand. The handheld unit 256 may have a 'head' or 'face' containing the transducer 250 and shaped to be positioned on a surface of a subject (e.g., against the skin). The sensor 208 may record properties of the probe 206, such as its rotational orientation or location in space. In some embodiments, the probe 206 may be accelerometer which may produce data used to determine movement of the probe 206. Although only a single sensor 240 is show in Figure 2, it is to be understood that the sensor 240 may represent a plurality of sensors positioned about the probe 240. The sensor 240 may be integral, such as contained within a housing of the probe 206, may be a separate component attached to an outside of a housing of the probe 206, or may be a combination of integral and combined. The sensor 240 may be located in a fixed position relative to the probe 206, so that by knowing a position of the sensor 240, a position of the probe 206 and imaging plane is also known.

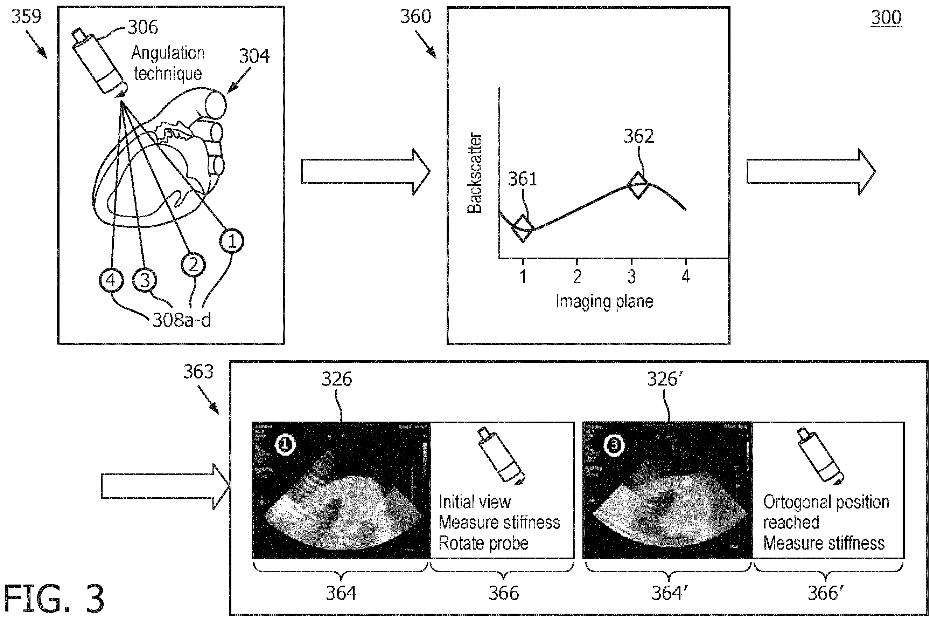

Figure 3 shows a method of collecting shear wave elastography measurements from complex anisotropic tissue in accordance with some examples of the present disclosure. In some embodiments, the method 300 may be implemented by the ultrasound system 102 of Figure 1 or the ultrasound system 200 of Figure 2. The method 300 includes block 359, which involves acquiring initial tissue measurements, block 360, which involves determining shear wave imaging planes, and block 363, which involves collecting shear wave measurements. The blocks in Figure 3 and their arrangement is illustrative only, and it is to be understood that the method 300 could involve additional or fewer steps, and that the steps may be repeated and/or performed in a different order. For example, a method according to the present disclosure may involve repeating the entire sequence shown in Figure 3 after moving the probe to a new location of interest about the tissue 304.

The method includes step 359, which involves collecting initial measurements from the tissue 304. The initial measurements may be used to determine properties of the anisotropic tissue 304 at a variety of measurement angles. A probe 306 acquires initial measurement data from an imaging plane which may be positioned at a plurality of different angles with respect to an orientation of the tissue 304. For example, the imaging plane may be rotated about several different axis with respect to the tissue 304 such as an axis perpendicular to a long axis of the fibers, an axis parallel to a face of the probe, etc. Figure 3 shows a probe 306 with four different imaging plane positions 308a-d. In some embodiments, the probe 306 may need to be physically rotated to collect a measurement from each imaging plane orientations 308a-d. As shown in Figure 3, the rotation may take the form of tilting the probe while maintaining contact with a surface of the subject, such that the imaging planes are rotated about an axis across the face of the probe. In some embodiments, the probe may be held stationary while beam steering is used to adjust the imaging plane position 308a-d. In some embodiments, the probe 306 may include a 2D transducer array, and the orientation of the imaging plane may be varied by selectively activating transducers of the array. In some embodiments, the probe 306 may acquire data from a plurality of imaging planes simultaneously, and may record data from each of the imaging plane orientations 308a-d.

An ultrasound system (e.g., system 102 of Figure 1 or 200 of Figure 2) may produce instructions for adjustment of the probe between orientations. The instructions may be displayed to a user (e.g., via feedback display 124 of Figure 1) or may be operated by a processor and/or controller of the system to automatically adjust between image plane orientations 308a-d. In some embodiments the image planes 308a-d are imaged sequentially, and a user of the system is prompted to adjust a current imaging plane until it matches the orientation of a target plane (e.g., the next imaging plane in the sequence). The system may record measurements from each of the image plane orientations 308a-d.

The system may generate a plurality of target orientations at which to take the initial measurements. In some embodiments, the target orientations may be separated by regular angular spacing (e.g., each target orientation is 5° separated from another target orientation). In other embodiments the spacing between orientations may be irregular. The system may track a current orientation of the probe 306 (e.g., with sensor 240 of Figure 2) and may determine when the current orientation of the probe 306 matches a target orientation. The system may prompt a user to record an initial measurement when the imaging plane matches a target orientation, or may automatically record an initial measurement when the imaging plane matches a target orientation. In some embodiments, the system may have a tolerance, and may indicate that a measurement is to be taken when the current orientation of the imaging plane is within the tolerance of the target orientation. Once a measurement is collected, the system may produce instructions to guide placement of the probe 306 to match a next of the target orientations.

The method 300 also includes step 360, determining shear wave imaging planes. An ultrasound system (e.g. system 102 of Figure 1, system 200 of Figure 2) may record the initial measurements from step 359. The measurements may be used to determine target orientations for shear wave elastography imaging. In some embodiments, the initial measurements may be acoustic measurements. Acoustic measurements may be derived from received echoes generated responsive to transmission of ultrasound signals. As shown in Figure 3, the initial measurements collected in step 359 were backscattering measurements, which may represent the angle between the imaging plane along which they were acquired and the orientation of fibers in the tissue. The backscattering measurements may be plotted versus the angle at which they were acquired with respect to a reference angle with respect to the tissue. For example, in some embodiments, the angle of the initial measurements may be measured with respect to the first initial measurement. In other examples, the angle of the initial measurements may be measured with respect to an anatomical feature of the subject. In some embodiments the angle of the measurements may be recorded by a user of the system. In some embodiments the angle at which the initial measurements were recorded may be measured by the system (e.g., based on sensors 240 of Figure 2). The plotted measurements may be displayed to a user of the system (e.g., on display 110 of Figure 1 or 210 of Figure 2), or may be used internally by a processor of the system. The plotted measurements may be a scatter plot and/or fitted curve.

A first imaging orientation 361 and a second imaging orientation 362 (e.g., a first imaging plane and a second imaging plane) may be determined based on the plotting of the initial measurements vs. the measurement angle. The angle of the first and second imaging orientations 361, 362 may represent the angle of fibers of the tissue with respect to the reference angle used to plot the initial measurements. The first and second imaging orientation may defined as angles corresponding to a maxima and a minima, respectively, of the backscattering (or other measurement type) in the plot. The maxima and minima may correspond to an orientation aligned with fibers of the tissue and an orientation orthogonal to the fibers. The maxima and minima may be global maxima and minima, or may be local maxima and minima within a specific region. The first and the second imaging orientations 361, 362 may be displayed to a user of the system and/or may be used to produce instructions for aligning an imaging plane of the probe 306 with the first imaging plane 361 and the second imaging plane 362.

In some embodiments, the step 360 may involve determining both the first and second imaging orientation 361, 362 from the initial measurements. In some embodiments, the step 360 may involve determining the only one of the first and second imaging orientation 361, 362 from the initial measurements, and may involve determining the other imaging orientation based on the determined orientation. For example, the first imaging orientation 361 may be determined by finding a minimum of the initial measurements, while the second imaging orientation 362 is determined by taking the plane orthogonal to the first imaging orientation 361.

The method 300 includes step 363, collecting shear wave measurements. The shear wave measurements may be collected at the first imaging plane 361 and the second imaging plane 362 determined in step 360. As shown in Figure 3, the collecting shear wave measurements are represented by image displays 326 and 326' which show images 364 and 364' collected at the first and second imaging planes 361, 362, respectively. The image displays 326 and 326' may, in some embodiments, implement the image display 126 of Figure 1. Although image displays 326 and 326' are shown, it is to be understood that the system may record shear wave measurements without necessarily producing shear wave images.

The system may produce instructions for collecting the shear wave measurements based on the determined first and second imaging planes 361, 362. The instructions for collecting the shear wave measurements may be similar to the instructions for collecting initial measurements described in regards to step 359. The system may set the first imaging plane 361 and the second imaging plane 362 as target planes. The system may provide feedback to a user (e.g., via orientation feedback display 124 of Figure 1) to adjust an imaging plane such that it aligns with the first and second imaging planes 361, 362. The system may determine a current orientation of the probe (e.g., with sensor 240 of Figure 2). The system may determine a difference between a current orientation of the probe 306 and the orientation of the first and second imaging planes 361, 362. In some embodiments, the system may prompt a user to record a shear wave elastography measurement when a current orientation of the probe 306 matches the first or second imaging plane 361, 362. In some embodiments, the system may automatically record a shear wave elastography measurement when the imaging plane matches the first or the second imaging plane 361, 362. The shear wave elastography measurements may be recorded by inducing a shear wave in the tissue (e.g., acoustic push pulse, mechanical vibration) and then tracking the propagation of the shear wave along a desired plane by transmitting spaced tracking pulses orthogonal to the desired plane. Magnitude of tissue displacement and/or propagation speed of the shear wave may be determined by analyzing received echoes generated responsive to the tracking pulses. The magnitude of tissue displacement and/or propagation speed of the shear wave may be used as shear wave elastography measurements or may be used to generate additional shear wave elastography measurements such as tissue stiffness. As discussed previously, the shear wave may propagate with different speeds in different directions in anisotropic tissue. For example, the shear wave may propagate at a first speed in a direction aligned with structures (e.g., fibers) of the tissue and at a second speed in a direction transverse to the structures of the tissue. Thus, different elastography measurements may be acquired at a same location by tracking the shear wave propagation in different directions.

The imaging displays 326 and 326' each show a respective image 364, 364' and a respective measurement orientation indicator 366, 366'. The imaging displays 326, 326' may be presented to a user of the system (e.g. via display 110 of Figure 1) and/or may be recorded in a memory of the system (e.g., memory 118 of Figure 1). The images 364, 364' may be shear wave elastography images recorded at the first and second imaging orientations 361, 362. The measurement orientation indicators 366, 366' may be graphic representations of the placement of the probe during the measurements representing in the respective imaging display 326, 326'.

The method 300 may be repeated for each location that the probe 306 is placed on the tissue 304. After the shear wave elastography measurements are collected in step 363, the system may prompt a user to reposition the probe 306 to a new location about the tissue 304. The system may then update to produce instructions for step 359 - collecting initial measurements, at the new location. The system may repeat this process for a number of locations about the tissue.

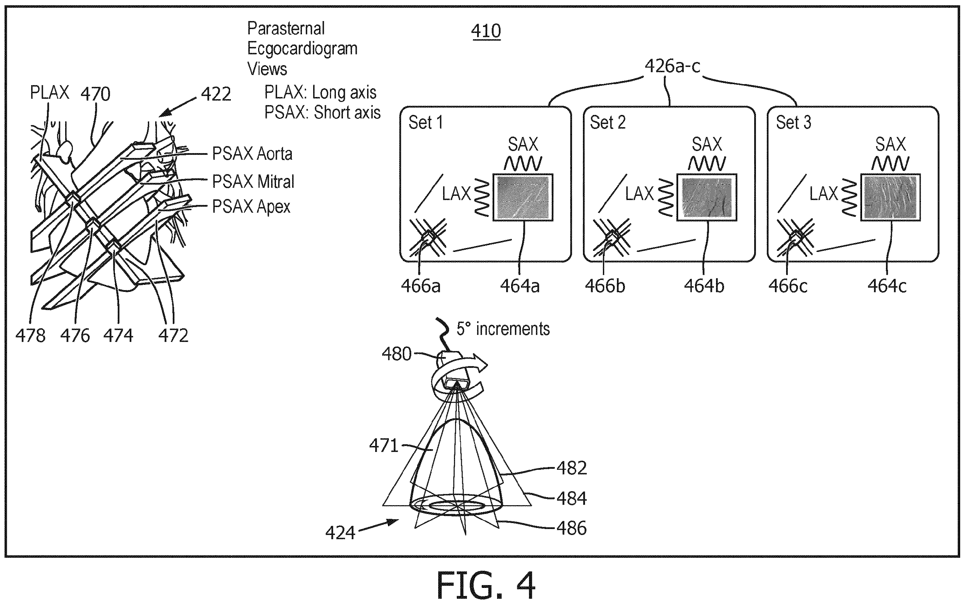

Figure 4 is a block diagram depicting an example display of an ultrasound system in accordance with some examples of the present disclosure. The display 410 may be implemented by display 110 of Figure 1 in some embodiments. The display shows a location feedback display 422, image displays 426a-c, and orientation feedback display 424. Although certain displays are shown in certain positions of the display 410, one of skill in the art would appreciate that more or less graphics could appear on the screen or that graphics on the screen could be rearranged without deviating from the disclosure. Similarly, while example layouts of displays 422, 424, and 426a-c are shown, the displays may contain more or less information, and may be include different elements, or different display characteristics (e.g., different shapes, colors, etc.) without deviating from the present disclosure. The display 410 may be updated in real-time and/or may show data saved on a memory of the system (e.g., memory 118 of Figure 1).

The display 410 includes a location feedback display 422 which displays placement of the imaging plane at various locations of interest about the tissue. The location feedback display 422 may include a tissue indicator 470, one or more imaging plane indicators 472, and location indicators including a target location indicator 474, a current location indicator 476, and a previous location indicator 478. Each of the location indicators 474-478 may represent the position of a location of interest on the tissue indicator 470. The tissue indicator 470 may be a graphical representation of an anisotropic tissue to be imaged (e.g., tissue 104 of Figure 1). The tissue indicator 470 may be realistic or representational depiction of the anisotropic tissue, and in some embodiments may show only an abstraction of the tissue (e.g., a rectangle). In the example of Figure 4, the tissue is cardiac tissue, and the tissue indicator 470 is a graphic of a heart.

The location feedback display 422 may also include one or more location indicators 474-478 displayed on top of the tissue indicator 470. The location indicators 474-478 may be graphical representations of probe placement at different locations of interest about the tissue. The location indicators may be stylized representations of a footprint of the probe, such as a wire frame. The location indicators may also be simple shapes (e.g., cubes, squares, circles, x's, etc.) to represent placement of the probe. The different location indicators 474-478 (discussed below) may have different appearances to distinguish them, such as different colors, textures, shading, line borders, etc.

The current location indicator 476 is a representation of the current location of the probe. The current location indicator 476 has a position on the tissue indicator 470 to represent the current location of the probe. In some embodiments, the location of the current indicator 476 on the tissue indicator 470 may be used to guide placement of the probe, based, for example, on the anatomy of the tissue. In some embodiments, the current location indicator 476 may be based on a measured current location of the probe, which may be determined, for example, based on a sensor of the probe (e.g., sensor 240 of Figure 2). In some embodiments, the current location indicator 476 may update in real-time.

The previous location indicator 478 may indicate previous locations at which shear wave elastography measurements were taken. The location feedback display 422 may display a plurality of previous location indicators 478. The system may also be configured to present a selected number of previous location indicators 478 (e.g., the most recent previous location). In some embodiments a user may select (e.g., via user interface 254 of Figure 2) a number of previous location indicators 478 to display.

The target location indicator 474 may represent a next location for shear wave elastography measurements. The system (e.g., ultrasound system 102 of Figure 1) may determine a location for a next set of shear wave elastography measurements to be collected at. The target location indicator 474 may be a graphical representation of that location. In some embodiments, the target location indicator 474 may only appear after a set of shear wave elastography measurements have been collected (e.g., by following the method 300 of Figure 3). The target location indicator 474 may indicate (e.g., by changing or colors) when the current location of the probe is aligned with the target location and prompt a user of the system to begin collecting a next set of shear wave measurements.

The location feedback display 422 also includes a plurality of imaging plane indicators 472. The imaging plane indicators 472 are graphical representations of shear wave elastography imaging planes (e.g., first imaging plate 361 and second imaging plane 362) displayed on the tissue indicator 470. The imaging plane indicators 472 may represent the first and second imaging planes for each of the location indicators 474-478. The locations of interest (represented by location indicators 474-478) may lie at the intersection of orthogonal pairs of the plane indicators 472. In the example of Figure 4, each of the locations of interest have been chosen such that a first imaging orientation of each of the locations of interest lies along a common axis. In particular, in the example of Figure 4, each of the locations of interest lies along a long axis of fibers of the tissue. The imaging plane indicators 472 may be displayed for previous imaging locations (e.g., at the previous location indicator 478), current imaging locations (e.g., at current location indicator 476), and/or at expected future imaging locations (e.g., target location indicator 474). The imaging plane indicators 472 may be displayed to align with the tissue indicator 470 in order to represent how the imaging planes align with the tissue anatomy.

The orientation feedback display 424 may be similar to the location feedback display 422, except the orientation feedback display 424 may guide an orientation of the imaging plane (e.g., rotation of the probe) rather than a location of the imaging plane (e.g., by placing the probe at different locations). The orientation feedback display 424 includes a probe indicator 480, a tissue indicator 471 and one or more orientation indicators, which may include a current orientation indicator 484, a previous orientation indicator 482, and/or a target orientation indicator 486. The tissue indicator 471 may be a graphical representation of anisotropic tissue that is being imaged. In some embodiments, the tissue indicator 471 may be a different kind of graphical representation than the tissue indicator 470 of the location feedback display 422. For example, the tissue indicator 471 may depict a depth or cross-section of the tissue, while the tissue indicator 470 may depict a surface and/or anatomy of the tissue.

The probe indicator 480 may depict a position of the probe in relation to the tissue. The probe indicator 480 may update in real-time to depict the current position of the probe. The probe indicator 480 may be a realistic depiction of the probe, or may be a simplified or schematic representation. The probe indicator 480 may include one or more instructions (e.g., an arrow) to represent adjustment of the probe.

The orientation feedback display includes orientation indicators 482-486. The orientation indicators 482-486 may be representations of an orientation (e.g., an angle) of an imaging plane of the probe (e.g., imaging plane 108 of Figure 1). The orientation indicators 482-486 may be displayed on the tissue indicator 471 and may represent the relative orientation of different imaging planes to each other and to the tissue. The orientation indicators 482-486 may represent a realistic shape of the imaging plane, or may be simplified views (e.g., a triangular orientation indicator may represent an imaging plane which is trapezoidal). The orientation indicators 482-486 may be different colors, or textures (e.g., dotted line borders) and the orientation feedback display 424 may include a legend to help distinguish the orientation indicators 482-486.

The current orientation indicator 484 is a graphical representation of a current orientation of an imaging plane of the probe (e.g., imaging plane 108 of Figure 1). The current orientation indicator 484 may update in real-time as the imaging plane is moved (e.g., by rotating the probe). In some embodiments, the current orientation indicator may reflect a measured orientation of the probe (e.g., measured with sensor 240 of Figure 2).

The previous orientation indicator 482 is a graphical representation of a previous imaging orientation of the imaging plane. One or more previous orientation indicators may be displayed on the tissue indicator 471. In some embodiments, only selected previous imaging orientations are indicated (e.g., only the most recent previous orientation).

The target orientation indicator 486 is a graphical representation of a next imaging orientation. The target orientation indicator may be a next orientation for collecting initial measurements (e.g., as in step 359 of Figure 3), or may be a first or second imaging plane for collecting shear wave elastography measurements (e.g., as in step 363 of Figure 3). In some embodiments there may be an indication of which type of measurement is to be performed (e.g., a different color of the target orientation indicator 486, a tone, a text display, etc.).

The system may prompt a user to adjust an orientation of the imaging plane such that the current orientation indicator 484 aligns with a target orientation indicator 486. The system may prompt a user to record an initial measurement such as a backscattering measurement (e.g., as in step 359 of Figure 3), or record a shear wave elastography measurement at a determined first or second imaging plane (e.g., as in step 363 of Figure 3). The system may prompt a user by, for example, changing a color of one or more of the indicators 482-486, sounding an alert or tone, and/or displaying a message on the display 410. In some embodiments, the system may automatically record a measurement when it detects that the current orientation of the imaging plane matches the target orientation. Once a measurement is recorded (e.g., to memory 118), the orientation feedback display 424 may update such that for example, the target orientation indicator 486 is marked as a previous orientation indicator 482, and a new target orientation indicator at a next orientation is displayed.

In some embodiments where the orientation of the imaging plane is adjustable without the need for user control (e.g., the probe includes a 2D array of transducers, the probe produces a plurality of imaging planes, etc.), the display 410 may not include an orientation feedback display 424. In some embodiments, the orientation feedback display 424 may still be displayed for reference even if user adjustment of the imaging plane is not required.

In some embodiments, the display 410 may alternate between presenting the location feedback display 422 and the orientation feedback display 424. For example, the location feedback display 422 may be displayed when the system is directing placement of the probe at a new location, while the orientation feedback display 424 may be displayed while the system is collecting initial measurement or shear wave measurements (e.g., as in method 300 of Figure 3).

In this manner, the location feedback display 422 may guide placement of the probe at a plurality of locations about a tissue, and the orientation feedback display 424 may guide rotation of the probe to a variety of imaging plane orientations. The system may operate and selectively display the feedback displays 422, 424 to guide placement of the probe at a location, collection of initial measurements at a plurality of angle, collection of shear wave measurements at a determined first and a second imaging plane, and placement of the probe at a new location.

The display 410 may also include imaging displays 426a-c. The imaging displays 426a-c may show representative images 464a-c taken at different positions and/or orientations. The images may be shear wave elastography images, or may be other forms of image, such as B-mode images. The imaging displays 426a-c may include an image 464a-c and a measurement position indicator 466a-c. The display indicators 464a-c shown in Figure 4 each include a representative image 464a-c, each taken at a different location at the tissue. The measurement position indicator 466a-c is a graphical representation of the location each image was recorded at. The imaging displays 426a-c may be similar to the imaging displays 326, 326' of Figure 3, except in Figure 4, the measurement position indicators 466a-c are measurement location indicators 466a-c instead of measurement orientation indicators 366a-c.

Since the image displays 426a-c are similar to each other, for the sake of brevity only one of the image displays 426a will be discussed in detail. However, it is to be understood that similar features may be included in each of the image displays 426a-c. Similarly, it is to be understood that the image displays 426a-c may vary slightly between each other, and that different options or features may exist in each display. For example, one display may have an indicator of the location it was taken at, while another has an indicator of the orientation of the image. The system may allow a user to select different image displays and/or configure the features of the displays (e.g., via user interface 254 of Figure 2).

Imaging display 426a includes an image 464a and a measurement location indicator 466a. The image 464a may be a representative image taken at the given location. In the example of Figure 4, the image 464a includes an orientation guide along the borders of the image 464a. One edge of the image is labeled as corresponding to a long axis of fibers of the tissue (e.g., LAX) while the other edge of the image 464a is labeled as corresponding to a short axis of the fibers (e.g., SAX). The orientation guide may be determined based on the initial measurements of the tissue (e.g., steps 359 and 360 of Figure 3). The measurement position indicator 466a may be a graphical representation of the measurement position that matches the graphical representation of the location feedback display 422. In some embodiments, the position indicator 466a may include target plane indictors (e.g., similar to the target plane indicators 472 of the location feedback display 422).

Figure 5 is an example report of an ultrasound system in accordance with some examples of the present disclosure. The report 500 may be generated by the system in response to the initial measurements and/or shear wave measurements collected by the system (e.g., in method 300 of Figure 3). The report 500 may be presented on a display (e.g. display 110 of Figure 1). The report may also be saved by the system (e.g., in memory 118 of Figure 1), printed, and/or sent to a viewing station separate from the ultrasound system (e.g., retrieved by a computer coupled to the ultrasound system 102 of Figure 1). The report 500 may include properties which are directly measured by the system and properties which are calculated from the measured properties.

In some embodiments, the report 500 may be a table, such as in the example of Figure 5. The report may also be a list, a graph, or other form of data organization known in the art. In the example report 500, the data in the table is organized into sets 590a-b, labeled in the first column. Each set 590a-b may correspond to a location of interest that the probe was positioned at for SWE measurements. The report 500 may include one or more properties 598a-d measured or calculated along the first imaging plane 592a-b and the second imaging plane 594a-b. The report 500 may also include one or more properties 598a-d calculated for composite shear wave measurements 596a-b. In the example report 500 of Figure 5, the composite measurements 596a-b may be determined from measurements at the first and second imaging plane 592a-b for each of the locations of interest 590a-b. As shown in Figure 5, the report 500 may include labels such as an indication of the plane at which the shear wave elastography image was taken. For example, the locations of interest 590a-b may be labeled with the cardiac view they correspond to.

In the example of Figure 5, set 590a corresponds to the parasternal short and long axis mitral views, while set 590b corresponds to the parasternal short and long axis apex views. Accordingly, the first imaging plane 592a may correspond to the parasternal long axis mitral view while the second imaging plane 594a may correspond to the parasternal short axis mitral view. In a similar manner, the first and second imaging plane 592b, 594b may correspond to the parasternal long and short axis apex view respectively.

The report 500 may include a measurement of thickness 598a of the tissue. The thickness 598a may be determined based on one or more images of the tissue. In some embodiments, the thickness 598a may be determined from the initial measurements, such as B-mode images of the tissue. The thickness 598a may be determined by applying image processing techniques (e.g., segmentation, machine learning) to the images to identify edges of the tissue. The thickness 598a may be calculated for each of the first and second imaging planes 592-594 at each of the locations of interest 590a-b or may be a single value for each location of interest 590a-b.

The report 500 may include an angle 598b that each measurement was taken at. The angle 598b may be the angle of the measurement for each of the first and second imaging planes 592-594 with respect to a reference angle. The angle 598b may be determined based on a measured orientation of the imaging plane during the measurement. In some embodiments, the angle 598b may be measured by a sensor in the probe (e.g., sensor 240 of Figure 2). The report may also include a measured shear wave speed 598c of the tissue at the given location and orientation. The shear wave speed 598c may be determined based on the shear wave elastography measurement at that location and orientation.

The report may also properties, such as stiffness 598d, which may be calculated based on one or more of the other properties 598a-c. The stiffness 598d may be calculated for each imaging plane 592-594 at each location of interest 590. In some embodiments, the stiffness 598d may be determined by a Lamb wave model. The Lamb wave model may use one or more measured properties such as thickness, angle, and/or shear wave speed to calculate the stiffness of the tissue.

As well as presenting data related to each measurement orientation within a set (e.g., parasternal short axis PSAX and parasternal long axis PLAX), the report 500 may include composite shear wave measurements or properties 596a-b which may be calculated based on a comparison between multiple individual measurements. In some examples the report 500 may include an average, a difference, and/or a ratio of the shear wave speeds between the different orientations. The report 500 may include composite shear wave measurements 596 calculated for certain of the properties 598a-d. For example, the report 500 may include a ratio between the stiffness 598d calculated at the first and second imaging planes 592, 594 at a given location of interest 590. Although only a comparison between first and second imaging planes 592-594 at each location of interest 590 is shown, the composite shear wave measurements 596a-b may also include properties calculated based on a comparison between measurements at different locations of interest 590a-b. For example, an average stiffness may be calculated for the first imaging plane (e.g., the parasternal long axis) at each of the locations of interest.

As described herein, a protocol for acquiring SWE measurements in anisotropic tissue may include scanning the tissue at a variety of orientations with respect to the tissue to acquire initial measurements (e.g., backscattering measurements). A minimum value of the initial measurements may indicate a first orientation to structures in the tissue (e.g., aligned with fibers in the tissue). A maximum value of the initial measurements may indicate an second orientation to structures in the tissue (e.g., perpendicular to the fibers in the tissue). The first orientation or the second orientation may be used to determine a first imaging plane. In some embodiments, the other of the first orientation or the second orientation may be used to determine a second imaging plane. In other embodiments, the second imaging plane may be selected by calculating a plane orthogonal to the first imaging plane.

SWE measurements may be acquired at an intersection of the first imaging plane and the second imaging plane. A first SWE measurement may be acquired at the intersection along the first imaging plane. To acquire the first SWE measurement, a shear wave may be induced (e.g., by a push pulse) in the tissue at the intersection and the propagation of the shear wave along the first imaging plane may be measured. A second SWE measurement may be acquired at the intersection along the second imaging plane. To acquire the second SWE measurement, a shear wave may be induced (e.g., by a push pulse) in the tissue at the intersection and the propagation of the shear wave along the second imaging plane may be measured.

Both the first and second SWE measurements may be provided (e.g., in a report). In some embodiments, the first and second SWE measurements may be used to generate a composite SWE measurements.

In some embodiments, the protocol described above may be repeated at different locations in the tissue.

In some applications, the systems and methods described herein may improve consistency and/or reliability of SWE measurements in anisotropic tissue. In some embodiments, the systems and methods may provide for ways of characterizing the anisotropy of tissues.

In various embodiments where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure it is noted that the various methods and devices described herein can be implemented in hardware, software and firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general purpose processing circuits which are programmed responsive to executable instruction to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, embodiments or processes described herein may be combined with one or more other examples, embodiments and/or processes or be separated and/or performed amongst separate devices or device portions in accordance with the present systems, devices and methods.

Finally, the above-discussion is intended to be merely illustrative of the present system and should not be construed as limiting the appended claims to any particular embodiment or group of embodiments. Thus, while the present system has been described in particular detail with reference to exemplary embodiments, it should also be appreciated that numerous modifications and alternative embodiments may be devised by those having ordinary skill in the art without departing from the scope of the present system as set forth in the claims that follow. Accordingly, the specification and drawings are to be regarded in an illustrative manner and are not intended to limit the scope of the appended claims.

## Claims

1. A method (300) of acquiring shear wave elastography measurements of anisotropic tissue, the method comprising:
acquiring initial measurements (359) from the anisotropic tissue by transmitting ultrasound beams toward the anisotropic tissue at a plurality of different angles with respect to an orientation of the anisotropic tissue;
determining a first imaging plane (361) at the angle associated with a maximum or a minimum value of the initial acoustic measurements, wherein the maximum value indicates a first orientation to a structure of the anisotropic tissue and the minimum value indicates a second orientation to the structure of the anisotropic tissue;
determining a second imaging plane (362);
generating a first shear wave at an intersection of the first imaging plane (361) and the second imaging plane (362);
acquiring a first shear wave elastography measurement by tracking the first shear wave propagation along the first imaging plane;
generating a second shear wave at the intersection of the first imaging plane and the second imaging plane;
acquiring a second shear wave elastography measurement by tracking the second shear wave propagation along the second imaging plane; and
generating a composite shear wave elastography measurement for the anisotropic tissue at the intersection of the first imaging plane and the second imaging plane based on the first and second shear wave elastography measurements.

2. The method of claim 1, wherein determining the second imaging plane includes determining the angle associated with the other of the maximum or the minimum value of the initial acoustic measurements.

3. The method of claim 1, wherein determining the second imaging plane includes determining an imaging plane orthogonal to the first imaging plane.

4. The method of claim 1, wherein the intersection of the first imaging plane and the second imaging plane is a first location of interest, the method further comprising acquiring an additional composite shear wave elastography measurement at a second location of interest at an intersection between the first imaging plane and an additional imaging plane spaced from the second imaging plane, wherein acquiring the additional composite shear wave elastography measurement comprises:
generating a third shear wave at the intersection of the first imaging plane and the additional imaging plane;
acquiring a third shear wave elastography measurement by tracking the third shear wave propagation along the first imaging plane;
generating a fourth shear wave at the intersection of the first imaging plane and the additional imaging plane;
acquiring a fourth shear wave elastography measurement by tracking the fourth shear wave propagation along the additional imaging plane; and
generating the additional composite shear wave elastography measurement based on the third and fourth shear wave elastography measurements.

5. The method of claim 4, wherein the anisotropic tissue is cardiac tissue, wherein the first imaging plane corresponds to a parasternal long axis view through of the cardiac tissue, and wherein the second and additional imaging planes correspond to two parasternal short axis views selected from the parasternal short axis aorta view, the parasternal short axis mitral view, and the parasternal short axis apex view.

6. The method of claim 5, further comprising generating a report of the shear wave elastography measurements for the cardiac tissue, wherein the report includes two or more different composite shear wave elastography measurements for each of the first and the second locations of interest.

7. The method of claim 1, wherein acquiring the initial measurements comprises recording backscatter coefficients from the anisotropic tissue at each of the plurality of different angles.

8. The method of claim 1, wherein generating the composite shear wave elastography measurement includes combining the first and second shear wave elastography measurements by computing a ratio, a sum, or a difference of the first and second shear wave elastography measurements.

9. The method of claim 1, further comprising displaying a graphical user interface configured to provide guidance for positioning the probe such that an imaging plane of the probe is aligned with the first imaging plane prior to acquiring the first shear wave elastography measurement and for repositioning the probe such that the imaging plane of the probe is aligned with the second imaging plane prior to acquiring the second shear wave elastography measurement.

10. The method of claim 1, wherein acquiring the initial measurements comprises scanning, using a 3D probe, a volumetric region including the anisotropic tissue to acquire a 3D dataset of backscatter measurements, and wherein acquiring the first and second shear wave elastography measurements comprises automatically steering, after determining the first imaging plane, the beams transmitted by the 3D probe to acquire the first and second shear wave elastography measurements.

11. An ultrasound system comprising:
a probe (256) configured to transmit ultrasound signals and acquire echoes responsive to the ultrasound signals to acquire measurements from an imaging plane; and
a processor (214,216) configured to:
cause the probe to acquire initial measurements from an anisotropic tissue at a plurality of angles with respect to an orientation of the anisotropic tissue;
determine a first imaging plane (361) at an angle associated with a maximum or minimum value of the initial measurements, wherein the maximum value indicates a first orientation to a structure of the anisotropic tissue and the minimum value indicates a second orientation to the structure of the anisotropic tissue;
determine a second imaging plane (362);
cause the probe to generate a first shear wave at an intersection of the first imaging plane and the second imaging plane;
acquire a first shear wave elastography measurement at the intersection of the first imaging plane and the second imaging plane by causing the probe to track the first shear wave's propagation along the first imaging plane;
cause the probe to generate a second shear wave at the intersection of the first imaging plane and the second imaging plane;
acquire a second shear wave elastography measurement at the intersection of the first imaging plane and the second imaging plane by causing the probe to track the second shear wave's propagation along the second imaging plane; and
generate a composite shear wave elastography measurement anisotropic tissue at the intersection of the first imaging plane and the second imaging plane based on the first and second shear wave elastography measurements.

12. The ultrasound system of claim 11, wherein the processor is further configured to generate location instructions for positioning the probe at a plurality of different locations of interest about the tissue, and wherein the ultrasound system ftuerh comprises a display configured to display feedback to guide the positioning of the probe at the plurality of different locations of interest based on the generated location instructions.

13. The ultrasound system of claim 11, further comprising sensors coupled to the probe and to the processor, the sensors configured to determine a current position and/or orientation of the probe and wherein the processor is further configured to generate orientation instructions are based, at least in part, on the determined current orientation.

14. The ultrasound system of claim 11, wherein the processor is further configured to determine a thickness of the tissue based on the recorded measurements and calculate a stiffness of the tissue based, at least in part, on the determined thickness and the shear wave elastography measurements.

15. The ultrasound system of claim 11, wherein the probe includes a 2D matrix array transducer, and wherein the processor is configured to automatically update the angle of the imaging plane to the first and the second imaging plane based on the determined first imaging plane and the orthogonal second imaging plane.

## Patentansprüche

1. Verfahren (300) zum Erfassen von Scherwellenelastographiemessungen anisotropem Gewebe, wobei das Verfahren Folgendes umfasst: erfassen erster Messungen (359) vom anisotropen Gewebe durch Senden von Ultraschallstrahlen in Richtung des anisotropen Gewebes in mehreren unterschiedlichen Winkeln in Bezug auf eine Ausrichtung des anisotropen Gewebes; bestimmen einer ersten Abbildungsebene (361) in dem Winkel, der einem Maximal- oder Minimalwert der anfänglichen akustischen Messungen zugeordnet ist, wobei der Maximalwert eine erste Ausrichtung zu einer Struktur des anisotropen Gewebes angibt und der Minimalwert eine zweite Ausrichtung dazu angibt Struktur des anisotropen Gewebes; bestimmen einer zweiten Bildebene (362); erzeugen einer ersten Scherwelle an einem Schnittpunkt der ersten Bildebene (361) und der zweiten Bildebene (362); erfassen einer ersten Scherwellen-Elastographiemessung durch Verfolgen der ersten Scherwellenausbreitung entlang der ersten Bildebene; erzeugen einer zweiten Scherwelle am Schnittpunkt der ersten Bildebene und der zweiten Bildebene; erfassen einer zweiten Scherwellen-Elastographiemessung durch Verfolgen der zweiten Scherwellenausbreitung entlang der zweiten Bildebene; und erzeugen einer zusammengesetzten Scherwellen-Elastographiemessung für das anisotrope Gewebe am Schnittpunkt der ersten Bildebene und der zweiten Bildebene auf der Grundlage der ersten und zweiten Scherwellen-Elastographiemessungen.

2. Verfahren nach Anspruch 1, wobei das Bestimmen der zweiten Bildebene das Bestimmen des Winkels umfasst, der mit dem anderen der maximalen oder minimalen Werte der anfänglichen akustischen Messungen verbunden ist.

3. Verfahren nach Anspruch 1, wobei das Bestimmen der zweiten Bildebene das Bestimmen einer Bildebene orthogonal zur ersten Bildebene umfasst.

4. Verfahren nach Anspruch 1, wobei der Schnittpunkt der ersten Bildebene und der zweiten Bildebene ein erster interessierender Ort ist, wobei das Verfahren weiterhin das Erfassen einer zusätzlichen zusammengesetzten Scherwellen-Elastographiemessung an einem zweiten interessierenden Ort an einem Schnittpunkt dazwischen umfasst die erste Bildebene und eine zusätzliche Bildebene, die von der zweiten Bildebene beabstandet ist, wobei das Erfassen der zusätzlichen Messung der zusammengesetzten Scherwellen-Elastographie Folgendes umfasst: erzeugen einer dritten Scherwelle am Schnittpunkt der ersten Bildebene und der zusätzlichen Bildebene; erfassen einer dritten Scherwellen-Elastographiemessung durch Verfolgen der dritten Scherwellenausbreitung entlang der ersten Bildebene; erzeugen einer vierten Scherwelle am Schnittpunkt der ersten Bildebene und der zusätzlichen Bildebene; erfassen einer vierten Scherwellen-Elastographiemessung durch Verfolgen der vierten Scherwellenausbreitung entlang der zusätzlichen Bildebene; und erzeugen der zusätzlichen zusammengesetzten Scherwellen-Elastographiemessung basierend auf den dritten und vierten Scherwellen-Elastographiemessungen.

5. Verfahren nach Anspruch 4, wobei das anisotrope Gewebe Herzgewebe ist, wobei die erste Bildebene einer parasternalen Langachsenansicht durch das Herzgewebe entspricht und wobei die zweite und zusätzliche Bildebenen zwei parasternalen Kurzachsenansichten entsprechen, ausgewählt aus der parasternalen Kurzachsen-Aorta-Ansicht, der parasternalen Kurzachsen-Mitralansicht und die parasternale Kurzachsen-Apexansicht.

6. Verfahren nach Anspruch 5, dass außerdem das Erstellen eines Berichts über die Scherwellenelastographiemessungen für das Herzgewebe umfasst, wobei der Bericht zwei oder mehr verschiedene zusammengesetzte Scherwellenelastographiemessungen für jeden der ersten und zweiten interessierenden Orte umfasst.

7. Verfahren nach Anspruch 1, wobei das Erfassen der Anfangsmessungen das Aufzeichnen von Rückstreukoeffizienten des anisotropen Gewebes bei jedem der mehreren unterschiedlichen Winkel umfasst.

8. Verfahren nach Anspruch 1, wobei das Erzeugen der zusammengesetzten Scherwellen-Elastographiemessung das Kombinieren der ersten und zweiten Scherwellen-Elastographiemessungen durch Berechnen eines Verhältnisses, einer Summe oder einer Differenz der ersten und zweiten Scherwellen-Elastographiemessungen umfasst.

9. Verfahren nach Anspruch 1, das außerdem das Anzeigen einer grafischen Benutzeroberfläche umfasst, die so konfiguriert ist, dass sie eine Anleitung zum Positionieren der Sonde bereitstellt, so dass eine Bildebene der Sonde mit der ersten Bildebene vor der Erfassung der ersten Scherwellen-Elastographiemessung und zur Neupositionierung ausgerichtet ist die Sonde so, dass die Abbildungsebene der Sonde mit der zweiten Abbildungsebene ausgerichtet ist, bevor die zweite Scherwellen-Elastographiemessung durchgeführt wird.

10. Verfahren nach Anspruch 1, wobei das Erfassen der Anfangsmessungen das Scannen eines volumetrischen Bereichs einschließlich des anisotropen Gewebes mit einer 3D-Sonde umfasst, um einen 3D-Datensatz von Rückstreumessungen zu erfassen, und wobei das Erfassen der ersten und zweiten Scherwellen-Elastographiemessungen das automatische Erfassen umfasst Lenken der von der 3D-Sonde gesendeten Strahlen nach der Bestimmung der ersten Bildebene, um die ersten und zweiten Scherwellen-Elastographiemessungen zu erfassen.

11. Ultraschallsystem, umfassend: eine Sonde (256), die zum Senden von Ultraschallsignalen und zum Erfassen von Echos als Reaktion auf die Ultraschallsignale konfiguriert ist, um Messungen von einer Bildebene zu erfassen; und einen Prozessor (214,216), der konfiguriert ist für: veranlassen, dass die Sonde Anfangsmessungen von einem anisotropen Gewebe in mehreren Winkeln in Bezug auf eine Ausrichtung des anisotropen Gewebes erfasst; bestimmen einer ersten Bildebene (361) in einem Winkel, der einem Maximal- oder Minimalwert der Anfangsmessungen zugeordnet ist, wobei der Maximalwert eine erste Ausrichtung zu einer Struktur des anisotropen Gewebes angibt und der Minimalwert eine zweite Ausrichtung zu der Struktur des anisotropen Gewebes angibt das anisotrope Gewebe; bestimmen einer zweiten Bildebene (362); veranlassen, dass die Sonde eine erste Scherwelle an einem Schnittpunkt der ersten Bildebene und der zweiten Bildebene erzeugt; erfassen einer ersten Scherwellen-Elastographiemessung am Schnittpunkt der ersten Bildebene und der zweiten Bildebene, indem die Sonde veranlasst wird, die Ausbreitung der ersten Scherwelle entlang der ersten Bildebene zu verfolgen; veranlassen, dass die Sonde am Schnittpunkt der ersten Bildebene und der zweiten Bildebene eine zweite Scherwelle erzeugt; erfassen einer zweiten Scherwellen-Elastographiemessung am Schnittpunkt der ersten Bildebene und der zweiten Bildebene, indem die Sonde veranlasst wird, die Ausbreitung der zweiten Scherwelle entlang der zweiten Bildebene zu verfolgen; und erzeugen einer zusammengesetzten Scherwellen-Elastographiemessung anisotropem Gewebe am Schnittpunkt der ersten Bildebene und der zweiten Bildebene auf der Grundlage der ersten und zweiten Scherwellen-Elastographiemessungen.

12. Ultraschallsystem nach Anspruch 11, wobei der Prozessor außerdem dazu konfiguriert ist, Positionsanweisungen zum Positionieren der Sonde an mehreren unterschiedlichen Orten von Interesse rund um das Gewebe zu generieren, und wobei das Ultraschallsystem außerdem eine Anzeige umfasst, die zur Anzeige von Rückmeldungen zur Führung konfiguriert ist die Positionierung der Sonde an mehreren verschiedenen interessierenden Orten auf der Grundlage der generierten Ortsanweisungen.

13. Ultraschallsystem nach Anspruch 11 umfasst ferner Sensoren, die mit der Sonde und dem Prozessor verbunden sind, wobei die Sensoren so konfiguriert sind, dass sie eine aktuelle Position und/oder Ausrichtung der Sonde bestimmen, und wobei der Prozessor ferner so konfiguriert ist, dass er Ausrichtungsanweisungen erzeugt, die zumindest teilweise auf der bestimmten aktuellen Ausrichtung basieren.

14. Ultraschallsystem nach Anspruch 11, wobei der Prozessor weiterhin dazu konfiguriert ist, eine Dicke des Gewebes auf Grundlage der aufgezeichneten Messungen zu bestimmen und eine Steifheit des Gewebes zumindest teilweise auf Grundlage der ermittelten Dicke und der Scherwellenelastographie zu berechnen Messungen.

15. Ultraschallsystem nach Anspruch 11, wobei die Sonde einen 2D-Matrix-Array-Wandler umfasst und wobei der Prozessor so konfiguriert ist, dass er den Winkel der Bildebene zur ersten und zweiten Bildebene basierend auf der ermittelten ersten Bildebene automatisch aktualisiert die orthogonale zweite Abbildungsebene.

## Revendications

1. Procédé (300) d'acquisition de mesures d'élastographie par ondes de cisaillement de tissu anisotrope, le procédé comprenant: acquérir des mesures initiales (359) à partir du tissu anisotrope en transmettant des faisceaux ultrasonores vers le tissu anisotrope sous une pluralité d'angles différents par rapport à une orientation du tissu anisotrope; déterminer un premier plan d'imagerie (361) à l'angle associé à une valeur maximale ou minimale des mesures acoustiques initiales, la valeur maximale indiquant une première orientation vers une structure du tissu anisotrope et la valeur minimale indiquant une seconde orientation vers la structure du tissu anisotrope; déterminer un deuxième plan d'imagerie (362); générer une première onde de cisaillement à une intersection du premier plan d'imagerie (361) et du deuxième plan d'imagerie (362); acquérir une première mesure d'élastographie par ondes de cisaillement en suivant la propagation de la première onde de cisaillement le long du premier plan d'imagerie; générer une seconde onde de cisaillement à l'intersection du premier plan d'imagerie et du deuxième plan d'imagerie; acquérir une seconde mesure d'élastographie par ondes de cisaillement en suivant la propagation de la seconde onde de cisaillement le long du second plan d'imagerie; et générer une mesure d'élastographie par ondes de cisaillement composite pour le tissu anisotrope à l'intersection du premier plan d'imagerie et du second plan d'imagerie sur la base des première et seconde mesures d'élastographie par ondes de cisaillement.

2. Procédé selon la revendication 1, dans lequel la détermination du deuxième plan d'imagerie comprend la détermination de l'angle associé à l'autre de la valeur maximale ou minimale des mesures acoustiques initiales.

3. Procédé selon la revendication 1, dans lequel la détermination du deuxième plan d'imagerie comprend la détermination d'un plan d'imagerie orthogonal au premier plan d'imagerie.

4. Procédé selon la revendication 1, dans lequel l'intersection du premier plan d'imagerie et du deuxième plan d'imagerie est un premier emplacement d'intérêt, le procédé comprenant en outre l'acquisition d'une mesure supplémentaire d'élastographie par ondes de cisaillement composites en un deuxième emplacement d'intérêt à une intersection entre le premier plan d'imagerie et un plan d'imagerie supplémentaire espacé du deuxième plan d'imagerie, l'acquisition de la mesure supplémentaire d'élastographie par ondes de cisaillement composite comprenant: générer une troisième onde de cisaillement à l'intersection du premier plan d'imagerie et du plan d'imagerie supplémentaire; acquérir une troisième mesure d'élastographie par ondes de cisaillement en suivant la propagation de la troisième onde de cisaillement le long du premier plan d'imagerie; générer une quatrième onde de cisaillement à l'intersection du premier plan d'imagerie et du plan d'imagerie supplémentaire; acquérir une quatrième mesure d'élastographie par ondes de cisaillement en suivant la propagation de la quatrième onde de cisaillement le long du plan d'imagerie supplémentaire; et générer la mesure d'élastographie par ondes de cisaillement composite supplémentaire sur la base des troisième et quatrième mesures d'élastographie par ondes de cisaillement.

5. Procédé selon la revendication 4, dans lequel le tissu anisotrope est du tissu cardiaque, dans lequel le premier plan d'imagerie correspond à une vue parasternale à axe long à travers le tissu cardiaque, et dans lequel le deuxième plan d'imagerie et les plans d'imagerie supplémentaires correspondent à deux vues parasternales à axe court sélectionnées parmi la vue parasternale à axe court de l'aorte, la vue parasternale à axe court mitral, et la vue du sommet parasternal à axe court.

6. Procédé selon la revendication 5, comprenant en outre la génération d'un rapport des mesures d'élastographie par ondes de cisaillement pour le tissu cardiaque, dans lequel le rapport comprend deux ou plusieurs mesures d'élastographie par ondes de cisaillement composites différentes pour chacun des premier et deuxièmes emplacements d'intérêt.

7. Procédé selon la revendication 1, dans lequel l'acquisition des mesures initiales comprend l'enregistrement des coefficients de rétrodiffusion à partir du tissu anisotrope à chacun de la pluralité d'angles différents.

8. Procédé selon la revendication 1, dans lequel la génération de la mesure d'élastographie par ondes de cisaillement composite comprend la combinaison des première et deuxièmes mesures d'élastographie par ondes de cisaillement en calculant un rapport, une somme ou une différence des première et deuxièmes mesures d'élastographie par ondes de cisaillement.

9. Procédé selon la revendication 1, comprenant en outre l'affichage d'une interface utilisateur graphique configurée pour fournir des indications sur le positionnement de la sonde de sorte qu'un plan d'imagerie de la sonde soit aligné avec le premier plan d'imagerie avant l'acquisition de la première mesure d'élastographie par ondes de cisaillement et pour repositionner la sonde de sorte que le plan d'imagerie de la sonde soit aligné avec le deuxième plan d'imagerie avant l'acquisition de la deuxième mesure d'élastographie par ondes de cisaillement.

10. Procédé selon la revendication 1, dans lequel l'acquisition des mesures initiales comprend le balayage, à l'aide d'une sonde 3D, d'une région volumétrique comprenant le tissu anisotrope pour acquérir un ensemble de données 3D de mesures de rétrodiffusion, et dans lequel l'acquisition des première et deuxième mesures d'élastographie par ondes de cisaillement comprend automatiquement piloter, après détermination du premier plan d'imagerie, les faisceaux transmis par la sonde 3D pour acquérir les première et deuxième mesures d'élastographie par ondes de cisaillement.

11. Système à ultrasons comprenant: une sonde (256) configurée pour transmettre des signaux ultrasonores et acquérir des échos en réponse aux signaux ultrasonores pour acquérir des mesures à partir d'un plan d'imagerie; et un processeur (214,216) configuré pour: amener la sonde à acquérir des mesures initiales à partir d'un tissu anisotrope sous une pluralité d'angles par rapport à une orientation du tissu anisotrope; déterminer un premier plan d'imagerie (361) selon un angle associé à une valeur maximale ou minimale des mesures initiales, la valeur maximale indiquant une première orientation vers une structure du tissu anisotrope et la valeur minimale indiquant une seconde orientation vers la structure du tissu anisotrope; déterminer un deuxième plan d'imagerie (362); amener la sonde à générer une première onde de cisaillement à une intersection du premier plan d'imagerie et du deuxième plan d'imagerie; acquérir une première mesure d'élastographie par ondes de cisaillement à l'intersection du premier plan d'imagerie et du deuxième plan d'imagerie en amenant la sonde à suivre la propagation de la première onde de cisaillement le long du premier plan d'imagerie; amener la sonde à générer une seconde onde de cisaillement à l'intersection du premier plan d'imagerie et du deuxième plan d'imagerie; acquérir une seconde mesure d'élastographie par ondes de cisaillement à l'intersection du premier plan d'imagerie et du second plan d'imagerie en amenant la sonde à suivre la propagation de la seconde onde de cisaillement le long du second plan d'imagerie; et générer un tissu anisotrope de mesure d'élastographie par ondes de cisaillement composite à l'intersection du premier plan d'imagerie et du second plan d'imagerie sur la base des première et seconde mesures d'élastographie par ondes de cisaillement.

12. Système à ultrasons selon la revendication 11, dans lequel le processeur est en outre configuré pour générer des instructions de localisation pour positionner la sonde à une pluralité de différents emplacements d'intérêt autour du tissu, et dans lequel le système à ultrasons comprend en outre un affichage configuré pour afficher un retour d'information pour guider le positionnement de la sonde au niveau de la pluralité de différents emplacements d'intérêt sur la base des instructions de localisation générées.

13. Système à ultrasons de la revendication 11, comprenant en outre des capteurs couplés à la sonde et au processeur, les capteurs étant configurés pour déterminer une position et/ou une orientation actuelle de la sonde et le processeur étant en outre configuré pour générer des instructions d'orientation basées, au moins en partie, sur l'orientation actuelle déterminée.

14. Système à ultrasons de la revendication 11, dans lequel le processeur est en outre configuré pour déterminer l'épaisseur du tissu sur la base des mesures enregistrées et calculer la rigidité du tissu sur la base, au moins en partie, de l'épaisseur déterminée et des mesures d'élastographie par ondes de cisaillement.

15. Système à ultrasons selon la revendication 11, dans lequel la sonde comprend un transducteur à réseau matriciel 2D, et dans lequel le processeur est configuré pour mettre à jour automatiquement l'angle du plan d'imagerie par rapport au premier et au deuxième plan d'imagerie sur la base du premier plan d'imagerie déterminé et le deuxième plan d'imagerie orthogonal.
